# FASCICULE DE BREVET EUROPEEN

(11) **EP 0 954 298 B1**
(45) Date de publication et mention de la délivrance du brevet: **08.03.2006**
(21) Numéro de dépôt: 97920619.0
(22) Date de dépôt: 03.04.1997
(51) Int. Cl.: A61K 31/185, A61P 15/10

(54) **DERIVES 2,5-DIHYDROXYBENZENESULFONIQUES POUR LE TRAITEMENT DE LA DYSFONCTION SEXUELLE**
2,5-DIHYDROXYBENZYLSULFONDERIVATE ZUR BEHANDLUNG VON SEXUELLEN STÖRUNGEN
2,5-DIHYDROXYBENZENESULFONIC DERIVATIVES FOR THE TREATMENT OF SEXUAL DYSFUNCTION

(30) Priorité: 03.04.1996 FR 9604182
(43) Date de publication de la demande: 10.11.1999
(60) Demande divisionnaire: 05112614.2
(73) Titulaire: LABORATORIOS DEL DR. ESTEVE, S.A., 08041 Barcelona (ES)
(72) Inventeur: ESTEVE-SOLER, José, E-08017 Barcelone (ES)
(74) Mandataire: Ahner, Francis
(86) Numéro de dépôt international: PCT/EP1997/001709
(87) Numéro de publication internationale: WO 1997/037647

(56) Documents cités:
- DE-A- 2 323 723
- ANN. PHARM. FR., vol. 30, no. 6, 1972, P3D016, pages 415-427, XP002022833 THOMAS, J. ET AL.: "Action du dobésilate de Calcium sur la résistence et la perméabilité capillaires et sur le temps de saignement et l'adhésivité plaquettaire modifiés par le dextran."
- TH¹RAPIE, vol. 24, 1969, pages 429-450, XP000615214 HUGUET ET AL.: "Action d'un hémostatique, la cyclonamine, sur la perméabilité et la résistance capillaires. Etude complémentaire."
- ARZNEIM. FORSCH., vol. 31, no. 6, 1981, P3D16, pages 962-965, XP002022724 SIM ET AL.: "The Evaluation of the Effects of the Venous Tonic 263-E on Capillary permeability in the Rabbit after Administration by Intradermal and Intravenous Routes"
- ARZNEIM. FORSCH., vol. 27, no. 5, 1977, pages 1073-1076, XP000615253 HLADOVEC: "Vasotropic Drugs - a Survey Based on a Unifying Concept of their Mechanism of Action"
- INNERE MEDIZIN , vol. 48, 1993, pages 140-149, XP000615251 STANDL: "Diabetische Mikroangiopathie"
- CHEMICAL ABSTRACTS, vol. 84, no. 1, 5 Janvier 1976 Columbus, Ohio, US; abstract no. 310k, MATHIEU ET AL.: "Effect of quinones and phenos on noradrenalinic hypertension in the rat." page 34; colonne 1; XP002022852 & BULL. SOC. R. SCI. LIÈGE, vol. 44, no. 3-4, 1975, pages 293-296, MATHIEU: "Effect of quinones and phenols on noradrenalinic hypertension in the rat."
- SAENZ DE TEJADA: "Impaired neurogenic and endothelium-mediated relaxation of penile smooth muscle from diabetic men with impotence" THE NEW ENGLAND JOURNAL OF MEDICINE, vol. 320, no. 16, pages 1025-1030,

## Description

La présente invention concerne l'utilisation des dérivés des acides dihydroxybenzènesulfoniques ainsi que leurs sels physiologiquement acceptables, pour la fabrication de médicaments destinés au traitement de la dysfonction sexuelle liée à un déficit du relâclement vasculaire endothélium, dépendant.

Des études décrivent des essais in-vivo dans lesquels le dobesilate de calcium, l'éthamsylate et/ou le persilate de bis(diéthylamine ont montré une activité restauratrice sur la résistance et/ou la perméabilité capillaire (ANN. PHARM. FR. vol. 30, n° 6, 1972 P3D016, pages 415-427 Thomas J et al. « Action du dobésilate de cacium sur la résistance et la perméabilité capillaires et sur le temps de saignement et l'adhésivité plaquettaire modifiés par le dextran » ; THERAPIE, vol. 24, 1969, pages 429-450, Huguet et al. « Action d'un hémostatique, la cyclonamine, sur la perméabilité et la résistance capillaires. Etude complémentaire »; ARZNEIM. FORSCH., vol. 31, n° 6, 1981, P3D16, pages 962-965 Sim et al. « The evaluation of the effects of the venous tonic 263-E on capillary permeability in the rabbit after intravenous routes ».

Il est également connu que le dobésilate de calcium et l'éthamsylate sont des agents vasotropiques utilisés dans le traitement notamment des phlébites, des thromboses, et ayant un effet protecteur sur l'endothélium (ARZNEIM. FORSCH., vol. 27, n° 5, 1977, pages 1073-1076, Hladovec « Vasotropic Drugs - a survey based on a unifying concept of ther mechanism of action ».

Il est également connu d'utiliser le dobésilate de calcium dans le traitement de la rétinopathie diabétique, qui fait partie de la microangiopathie diabétique (INNERE MEDIZIN, vol. 48, 1993, pages 140-149, Standl « Diabetische mikroangiopathie ».

Par ailleurs, le dihydroxybenzènedisulfonate de calcium et le persilate de bis(diéthylamine) sont connus pour diminuer la perméabilité des vaisseaux et leur effet protecteur sur les vaisseaux (DE 23 23 723 A).

Des études récentes ont démontré que le Dobésilate de calcium, l'Ethamsylate et le Persilate exercent des effets au niveau de l'Endothélium dans le sens de faciliter le relâchement vasculaire endothélium-dépendant. Cet effet s'observe tant chez les animaux normaux comme chez ceux à qui on a produit expérimentalement un vieillissement vasculaire moyennant l'administration de doses élevées de vitamine D₂. Cette activité du Dobésilate de calcium, de l'Ethamsylate et du Persilate peut se traduire chez l'homme par des effets d'utilité thérapeutique. En particulier, on a démontré que l'érection du pénis est modulée par l'oxyde nitrique produit au niveau endothélial (A.L Burnett et al., Science, 1992, 257:401-403 ; J. Rajfer et al., N. Engl. J. Med., 1992, 326: 90-94; Editorial, Lancet, 1992, 340: 882-883) et que dans les circonstances où la fonction endothéliale est altérée, comme dans les hyperlipidémies (K. Kugiyama et al., Nature, 1990, 344 : 160-162), la correction de l'altération normalise la fonction érectile, mesurée avec précision pendant le sommeil nocturne (J.-B. Kostis et al., J. Clin. Pharmacol., 1994, 34 : 989-996 ; R.C. Rosen, "The Pharmacology of Sexual Function and Dysfunction", J. Bancroft ed. Elsevier Sc., 1995, pages 277-287). La normalisation de la fonction endothéliale obtenue avec le Dobésilate de calcium, l'Ethamsylate et le Persilate peut représenter une approche thérapeutique totalement nouvelle au problème de l'impuissance (I. Saenz de Tejada et al., N. Engl. J. Med., 1989, 320: 1025-1030) tant chez les patients avec des troubles vasculaires de causes diverses (diabète, artério-sclérose, etc) comme chez les patients où on ne peut détecter qu'un trouble fonctionnel.

D'autre part, l'effet normalisateur de la fonction Endothéliale peut offrir également des opportunités thérapeutiques dans des processus de spasmes vasculaires, dans les complications du diabète (W. Durante et al., Br. J. Pharmacol., 1988, 94 : 463-468) et dans tous les troubles, l'éjaculation précoce inclue (E M. Hull et al., Neuropharmacology, 1994, 33 : 1499-1504), où un déficit de formation de l'oxyde nitrique pour l'endothélium vasculaire apparait évident.

Les composés préconisés dans le cadre de la présente invention répondent à la formule générale I : 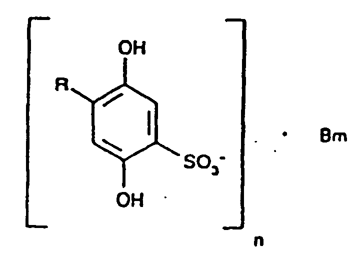
dans laquelle :
- R: représente un atome d'hydrogène (H) on un groupement sulfonique (SO₃-)
- B: représente un ion calcium (Ca++ ) ou un groupement diéthylamine [H₂N+ (C₂H₅)₂]
- n: représente 1 ou 2
- m: représente 1 ou 2

Les composés des exemples que l'on indique ci-après se préparent d'après des procédés décrits antérieurement :

### Exemple 1 :

2,5-Dihydroxybenzène sulfonate de calcium (Dobésilate de calcium). "The Merck Index", 11th edition, Merck & Co., Rahway, N.J., USA, 1989.

### Exemple 2 :

2,5-Dihydroxybenzène sulfonate de diéthylamine (Ethamsilate). "The Merck Index", 11th edition, Merck & Co., R Rahway, N.J., USA, 1989.

### Exemple 3 :

2,5-Dihydroxybenzène- 1,4-disulfonate de bis (diéthylamine) (Persilate de bis (diéthylamine). Brevet français FR 73/17709 (numéro de publication 2.201.888)

Pour étudier l'effet normalisateur de la fonction endothéliale des produits de la présente invention, on a réalisé différentes études "in vitro" et "in vivo". On décrit ci-après les résultats obtenus, avec un des produits de la présente invention, le Dobésilate de calcium, en démontrant qu'il possède une activité relâchante dépendant de l'Endothélium.

Les études ont été réalisées en utilisant les aortes isolées de lapins males, d'après les techniques décrites par A. Quintana et al. (Europ. J. Pharmacol, 1978, 53: 113-116) et par le groupe de l'Université d'Edimburg (Pharmacological Experiments on Isolated Preparations, Edimburg, Livingstone, 1970). Les aortes isolées sans l'Endothélium se préparent d'après la technique décrite par R. Furchgott et al. (J. Cardiol. Pharmacol., 1984, 6: S336-S343).

### A.- ETUDES "IN VITRO"

1) Effet sur la contraction de l'aorte, maintenu par 10⁻⁶M de noradrénaline.
   Le Dobésilate de calcium relâche la contraction de noradrénaline de façon dépendante de la concentration (entre 10⁻⁴ M et 10⁻¹¹ M). Le relâchement maximum a été de 70 %.
2) Effet sur la tension basale dans les artères aortes avec et sans Endothélium (tension basale = 2 grammes).
   Le Dobésilate de calcium à des concentrations entre 10⁻⁸ M et 10⁻⁴, relâche de façon dépendante de la dose les aortes soumises à une tension basale de 2 grammes. Le relâchement maximum a été de 44 % pour les artères avec de l'Endothélium et de 48 % pour les artères sans Endothélium.
3) Effet sur la courbe concentration-effet de noradrénaline.
   Dans des artères avec l'Endothélium intact, le Dobésilate de calcium à la concentration de 10⁻⁶ M, inhibe un 44 % la contraction obtenue avec 10⁻⁴ M de noradrénaline. Par contre, avec des artères sans Endothélium, il n'y a aucune modification de la courbe.

### B.- ETUDES "IN VIVO"

On a étudié l'effet protecteur de l'Endothélium par le Dobésilate de calcium chez le lapin.

Pour cela on endommage l'Endothélium artériel chez les lapins par un traitement journalier avec une surdose de vitamine D₂ et on travaille avec trois groupes d'animaux :

### O- Contrôle

I - Hypervitaminose D₂
II - Hypervitaminose D₂ + Dobésilate de calcium 50 mg/kg/jour.

On effectue les études avec les artères provenant des lapins traités en mesurant l'effet sur la contraction de l'aorte maintenu par 10⁻⁶ M de noradrénaline.

Le Dobésilate de calcium (entre 10⁻¹¹ M et 10⁻⁵ M) relâche de façon dose-dépendante la contraction due à la noradrénaline. Le relâchement moindre correspond au groupe d'hypervitaminose D₂ (groupe 1) alors que le relâchement maximum s'obtient avec les aortes du groupe traité avec le Dobésilate de calcium (groupe II).

Le relâchement maximum obtenu pour chaque groupe a été le suivant :
Groupe O (contrôle) : 69 %
Groupe I (hypervitaminose D₂ : 52 %M
Groupe II (hypervitaminose D₂ + Dobésilate de Calcium) : 100 %

Les résultats obtenus démontrent que le Dobésilate de calcium agit en potentialisant la synthèse et/ou la libération ou bien en diminuant la destruction d'un facteur relâchant qui dépend de l'Endothélium vasculaire et qui est probablement libéré par la noradrénaline à travers son action sur les réceptions α₂ adrénergiques.

En thérapeutique humaine, la dose d'administration est bien sûr fonction de la gravité de affection à traiter. Elle sera généralement comprise entre environ 0,5 et environ 2g/jour. Les dérivés de l'invention seront, par exemple, administrés sous forme de gélules ou de comprimés. On indiquera ci-après, à titre d'exemples, deux formes galéniques particulières.

### Exemple de formule par gélule :

| | |
|---|---|
| Dobésilate de calcium | 0,500 g |
| Cellulose | 0,023 g |
| Estéarate de magnésium | 0,007 g |
| Dioxyde de silice colloïdale | 0,005 g |
| | 0,535 g |

### Exemple de formule par comprimé :

| | |
|---|---|
| Dobésilate de calcium | 0,2500 g |
| Amidon de maïs | 0,0650 g |
| Lactose | 0,0520 g |
| Povidone K-30 | 0,0175 g |
| Acide citrique monohydrate | 0,0125 g |
| Estéarate de magnésium | 0,0020 g |
| Bisulfite de sodium | 0,0010 g |
| | 0,4000 g |

Compte tenu des intéressantes propriétés pharmacologiques attachées aux composés de formule générale I, la présente invention s'étend à l'utilisation de ces composés pour la fabrication de médicaments destinés au traitement de la dysfonction sexuelle liée à un déficit de relâchement vasculaire endothélium-dépendant.

## Revendications

1. Utilisation des dérivés de 2,5-dihydroxybenzènesulfoniques de formule générale 1 : 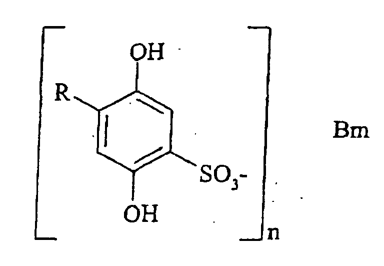
dans laquelle :
R représente H ou SO₃⁻
B représente Ca⁺⁺ ou H₂N⁺(C₂H₅)₂
n représente 1 ou 2
m représente 1 ou 2
pour la fabrication de médicaments destinés au traitement de la dysfonction sexuelle liée à un déficit du relâchement vasculaire endothélium-dépendant.

2. Utilisation selon la revendication 1, **caractérisée en ce que** le dérivé de formule générale 1 est le 2,5-dihydroxybenzènesulfonate de calcium [DOBÉSILATE DE CALCIUM].

3. Utilisation selon la revendication 1, **caractérisée en ce que** le dérivé de formule générale I est le 2,5-dihydroxybenzènesulfonate de diéthylamine [ETHAMSYLATE].

4. Utilisation selon la revendication 1, **caractérisée en ce que** le dérivé de formule générale 1 est le 2,5 dihydroxybenzène-1,4-disulfonate de bis (diéthylamine) [PERSILATE].

## Claims

1. Use of the 2,5-dihydroxybenzenesulphonic derivatives of general formula I: 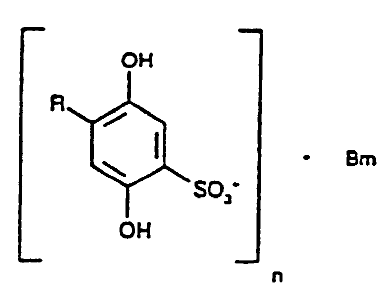
in which:
R represents H or SO₃⁻
B represents Ca⁺⁺ or H₂N⁺(C₂H₅)₂
n represents 1 or 2
m represents 1 or 2
for the manufacture of medicaments intended for the treatment of sexual dysfunction related to a deficiency in endothelium-dependent vascular relaxation.

2. Use according to Claim 1, **characterized in that** the derivative of general formula I is calcium 2,5-dihydroxybenzenesulphonate [CALCIUM DOBESILATE].

3. Use according to Claim 1, **characterized in that** the derivative of general formula I is diethylamine 2,5-dihydroxybenzenesulphonate [ETHAMSYLATE].

4. Use according to Claim 1, **characterized in that** the derivative of general formula I is bis(diethylamine) 2,5-dihydroxybenzene-1,4-disulphonate [PERSILATE].

## Patentansprüche

1. Verwendung von 2,5-Dihydroxybenzolsulfonat-Derivaten der allgemeinen Formel I: 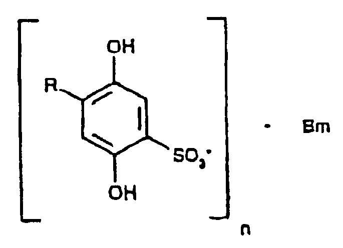
in der:
R für H oder SO₃⁻ steht;
B für Ca⁺⁺ oder H₂N⁺(C₂H₅)₂ steht;
n für 1 oder 2 steht;
m für 1 oder 2 steht;
für die Herstellung von Medikamenten, die zur Behandlung der sexuellen Dysfunktion bestimmt sind, die mit einem Defizit der Endothel-abhängigen vaskulären Relaxation verbunden ist.

2. Verwendung nach Anspruch 1, **dadurch gekennzeichnet, dass** das Derivat der allgemeinen Formel I Calcium-2,5-dihydroxybenzolsulfonat [CALCIUM DOBELISAT] ist.

3. Verwendung nach Anspruch 1, **dadurch gekennzeichnet, dass** das Derivat der allgemeinen Formel I Diethylamin-2,5-dihydroxybenzolsulfonat [ETHAMSYLAT] ist.

4. Verwendung nach Anspruch 1, **dadurch gekennzeichnet, dass** das Derivat der allgemeinen Formel I Bis(diethylamin)-2,5-dihydroxybenzol-1,4-disulfonat [PERSILAT] ist.
